# EUROPEAN PATENT SPECIFICATION

(11) **EP 2 882 454 B1**
(45) Date of publication and mention of the grant of the patent: **10.10.2018**
(21) Application number: 13748450.7
(22) Date of filing: 06.08.2013
(51) Int. Cl.: A61K 39/395

(54) **COMBINATION THERAPY FOR THE TREATMENT OF GLIOBLASTOMA**
KOMBINATIONSTHERAPIE ZUR BEHANDLUNG VON GLIOBLASTOMEN
POLYTHÉRAPIE POUR LE TRAITEMENT D'UN GLIOBLASTOME

(30) Priority: 07.08.2012 US 201261680672 P; 16.10.2012 US 201261714438 P; 05.02.2013 US 201361760763 P
(43) Date of publication of application: 17.06.2015
(60) Divisional application: 18178511.4
(73) Proprietor: F. Hoffmann-La Roche AG, 4070 Basel (CH)
(72) Inventor: DAS, Asha, South San Francisco, California 94080 (US); GUIJARRO, Ana Maria Abajo, 4070 Basel (CH); ABREY, Lauren, 4070 Basel (CH); GARCIA, Josep, 4070 Basel (CH); HILTON, Magalie, 4070 Basel (CH); KERLOEGUEN, Yannick, 4070 Basel (CH); MOORE, Nicola, 4070 Basel (CH)
(74) Representative: Denison, Christopher Marcus
(86) International application number: PCT/US2013/053841
(87) International publication number: WO 2014/025813

(56) References cited:
- TW-A- 201 138 819
- US-A1- 2010 266 589
- WICK W ET AL: "Chemotherapy of gliomas", ONKOLOGE 2011 SPRINGER VERLAG DEU, vol. 17, no. 1, January 2011 (2011-01), pages 44-54, XP002714438, ISSN: 0947-8965
- LAI A ET AL: "Phase II Pilot Study of Bevacizumab in Combination with Temozolomide and Regional Radiation Therapy for Up-Front Treatment of Patients With Newly Diagnosed Glioblastoma Multiforme: Interim Analysis of Safety and Tolerability", INTERNATIONAL JOURNAL OF RADIATION: ONCOLOGY BIOLOGY PHYSICS, PERGAMON PRESS, USA, vol. 71, no. 5, 1 August 2008 (2008-08-01) , pages 1372-1380, XP022939501, ISSN: 0360-3016, DOI: 10.1016/J.IJROBP.2007.11.068 [retrieved on 2008-03-20]

## Description

### FIELD OF THE INVENTION

This invention concerns in general treatment of diseases and pathological conditions with anti-VEGF antibodies. More specifically, the invention concerns the treatment of human patients susceptible to or diagnosed with glioblastoma using an anti-VEGF antibody, in combination with one or more additional anti-tumor therapeutic agents.

### BACKGROUND

Gliomas account for 81% of all malignant brain and CNS tumors. Glioblastoma - World Health Organization (WHO) grade IV astrocytoma - accounts for 60% to 70% of malignant gliomas and remains the most aggressive sub-type of glioma. It occurs mostly in adults (median age at diagnosis: 64 years) and its incidence is estimated to be 3.05/100'000 in the United States and less than 2/100'000 in Europe. With 1- and 5-year overall survival of 29% and 3%, respectively, the prognosis of glioblastoma remains particularly poor (Central Brain Tumor Registry of the United States (2005),(CBTRUS; http://www.cbtrus.org).

Although some progress has been made in the treatment of glioblastoma, this disease faces a highly unmet medical need with limited treatment options. In particular, bevacizumab (Avastin®), a monoclonal antibody targeted against the pro-angiogenic vascular endothelial growth factor (VEGF), holds significant therapeutic potential.

Lai et al. (2008) International Journal of Radiation: Oncolcogy Biology Physics 71(5): 1372-1380 reports interim analysis of a phase II pilot study of bevacizumab in combination with temozolomide and regional radiation therapy for up-front treatment of patients with newly diagnosed glioblastoma multiforme.

### SUMMARY OF THE INVENTION

The present invention provides anti-VEGF antibodies for use as defined in the claims in methods of treating patients diagnosed with glioblastoma, including patients newly diagnosed with glioblastoma.

The methods comprise administering to said patient a therapy comprising an effective amount of an anti-VEGF antibody, an effective amount of a chemotherapeutic, and radiotherapy wherein said treatment prolongs said patient's median progression-free survival time as compared to a glioblastoma patient receiving said chemotherapeutic without the anti-VEGF antibody. In one embodiment, the patient has a WHO performance status of ≤2. In some embodiments, the chemotherapeutic is temozolomide. In some embodiments, the effective amount of the temozolomide is mg/m² administered orally. In some embodiments, the effective amount of the temozolomide is 200 mg/m² administered orally. In some embodiments, the anti-VEGF antibody binds the A4.6.1 epitope. In some embodiments, the anti-VEGF antibody is bevacizumab. In some embodiments, the anti-VEGF antibody comprises a variable heavy chain (VH) and a variable light chain (VL), wherein said VH has an amino acid sequence of SEQ ID NO:2 and said VL has an amino acid sequence of SEQ ID NO:1. In some embodiments, the effective amount of said anti-VEGF antibody is 10 mg/kg intravenously every two weeks, administered, for example, initially intravenously over 90 minutes, with subsequent infusions over 60 minutes and then 30 minutes. In some embodiments, the effective amount of said anti-VEGF antibody is 15 mg/kg intravenously every three weeks administered, for example, initially intravenously over 90 minutes, with subsequent infusions over 60 minutes and then 30 minutes. In the methods described above, the anti-VEGF antibody is administered first to said patient at the first cycle and then subsequent administrations of said anti-VEGF antibody are either prior to or after said chemotherapeutic. In another embodiment, the anti-VEGF antibody is administered concurrently with said chemotherapeutic and radiotherapy. In some embodiments, administration of steroid to the patient is discontinued.

In the methods described above for treating a patient diagnosed with a glioblastoma, the median progression-free survival time is prolonged by about 4.4 months with a hazard ratio (HR) equal to 0.64, as compared to a glioblastoma patient receiving said chemotherapeutic without the anti-VEGF antibody . In another embodiment, the median progression-free survival time is prolonged by at least 4 months or greater with a hazard ratio (HR) equal to 0.64, as compared to a glioblastoma patient receiving said chemotherapeutic without the anti-VEGF antibody . In another embodiment, the median progression-free survival time is prolonged by at least 4 months or greater with a hazard ratio (HR) from about 0.55 to about 0.74, as compared to a glioblastoma patient receiving said chemotherapeutic without the anti-VEGF antibody . In another embodiment, the median progression-free survival time is prolonged by about 4.4 months with a hazard ratio (HR) from about 0.55 to about 0.74, as compared to a glioblastoma patient receiving said chemotherapeutic without the anti-VEGF antibody . In yet another embodiment in the methods described above, the patient is less than 65 years old. In yet another embodiment in the methods described above, the patient is equal to or greater than 65 years old. In one embodiment in the methods described above, the patient has a treatment free interval (TFI) of about 4 weeks.

Another embodiment of the disclosure provides kits comprising an anti-VEGF antibody binding essentially to epitope A4.6.1, a chemotherapeutic and a package insert or label with instructions to treat a patient diagnosed with glioblastoma comprising administering to said patient an effective amount of an anti-VEGF antibody and a chemotherapeutic, wherein said treatment prolongs said patient's median progression-free survival time as compared to a glioblastoma patient receiving said chemotherapeutic without the anti-VEGF antibody . In another embodiment of the kit described above, the anti-VEGF antibody is bevacizumab and said chemotherapeutic is temozolomide.

### BRIEF DESCRIPTION OF THE DRAWINGS

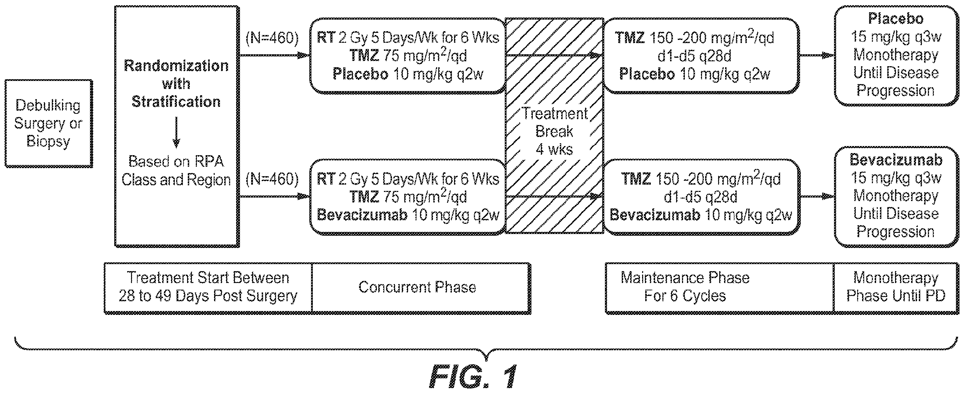

Figure 1 shows the two-arm Phase III study design treatment sequence as disclosed in more detail in Example 1. Study treatment started between 4 and 7 weeks after debulking surgery or biopsy of the glioblastoma and included 3 different phases: A Concurrent Phase during which 10 mg/kg bevacizumab or placebo was administered every two weeks in combination with temozolomide and radiotherapy followed by a treatment break of 28 days, a Maintenance Phase during which 10 mg/kg bevacizumab or placebo was administered every two weeks in combination with temozolomide, and a Monotherapy Phase during which 15 mg/kg bevacizumab or placebo was administered every three weeks until disease progression.

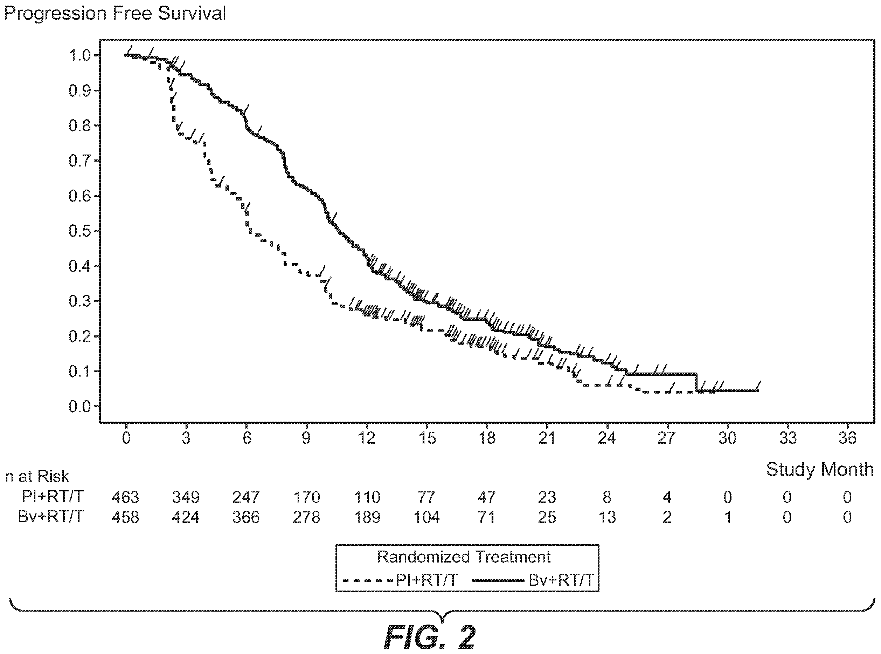

Figure 2 shows the Kaplan Meier Curves for PFS from the phase III AVAglio trial.

### DETAILED DESCRIPTION OF EMBODIMENTS OF THE INVENTION

### DEFINITIONS

An "anti-angiogenesis agent" or "angiogenesis inhibitor" refers to a small molecular weight substance, a polynucleotide, a polypeptide, an isolated protein, a recombinant protein, an antibody, or conjugates or fusion proteins thereof, that inhibits angiogenesis, vasculogenesis, or undesirable vascular permeability, either directly or indirectly. It should be understood that the anti-angiogenesis agent includes those agents that bind and block the angiogenic activity of the angiogenic factor or its receptor. For example, an anti-angiogenesis agent is an antibody or other antagonist to an angiogenic agent as defined throughout the specification or known in the art, e.g., but are not limited to, antibodies to VEGF-A or to the VEGF-A receptor (e.g., KDR receptor or Flt-1 receptor), VEGF-trap, anti-PDGFR inhibitors such as Gleevec™ (Imatinib Mesylate). Anti-angiogensis agents also include native angiogenesis inhibitors, e.g., angiostatin, endostatin, etc. See, e.g., Klagsbrun and D'Amore, Annu. Rev. Physiol., 53:217-39 (1991); Streit and Detmar, Oncogene, 22:3172-3179 (2003) (e.g., Table 3 listing anti-angiogenic therapy in malignant melanoma); Ferrara & Alitalo, Nature Medicine 5:1359-1364 (1999); Tonini et al., Oncogene, 22:6549-6556 (2003) (e.g., Table 2 listing known antiangiogenic factors); and Sato. Int. J. Clin. Oncol., 8:200-206 (2003) (e.g., Table 1 lists anti-angiogenic agents used in clinical trials).

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

The term "ascites" or abdominal ascites refers to fluid that has accumulated in the abdomen in excess amount. Ascitic fluid often contains free-floating cancer cells which have broken off from the cancerous growths. The presentation of abdominal ascites typically indicates a more symptomatic disease and a poorer outcome as compared to those patients who do not have abdominal ascites.

The term "bevacizumab" refers to a recombinant humanized anti-VEGF monoclonal antibody generated according to Presta et al. (1997) Cancer Res. 57:4593-4599, also known as "rhuMAb VEGF" or "AVASTIN®". It comprises mutated human IgGl framework regions and antigen-binding complementarity-determining regions from the murine anti-human VEGF monoclonal antibody A.4.6.1 that blocks binding of human VEGF to its receptors. Approximately 93% of the amino acid sequence of bevacizumab, including most of the framework regions, is derived from human IgGl, and about 7% of the sequence is derived from the murine antibody A4.6.1. bevacizumab binds to the same epitope as the monoclonal anti-VEGF antibody A4.6.1 produced by hybridoma ATCC HB 10709.

"The terms "cancer" and "cancerous" refer to or describe the physiological condition in mammals that is typically characterized by unregulated cell growth. Included in this definition are benign and malignant cancers as well as dormant tumors or micrometastatses. Examples of cancer include but are not limited to, carcinoma, lymphoma, blastoma, sarcoma, and leukemia. More particular examples of such cancers include but are not limited to, glioblastoma (GBM), including, e.g., proneural GBM, neural GBM, classical GBM and mesenchymal GBM. Other cancers include, for example, breast cancer, squamous cell cancer, lung cancer (including small-cell lung cancer, non-small cell lung cancer, adenocarcinoma of the lung, and squamous carcinoma of the lung), cancer of the peritoneum, hepatocellular cancer, gastric or stomach cancer (including gastrointestinal cancer), pancreatic cancer, ovarian cancer, cervical cancer, liver cancer, bladder cancer, hepatoma, colon cancer, colorectal cancer, endometrial or uterine carcinoma, salivary gland carcinoma, kidney or renal cancer, liver cancer, prostate cancer, vulval cancer, thyroid cancer, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome.

A "chemotherapeutic agent" is a chemical compound useful in the treatment of cancer. Examples of chemotherapeutic agents include is a chemical compound useful in the treatment of cancer. Examples of chemotherapeutic agents include alkylating agents, such as, for example, temozolomide, the imidazotetrazine derivative of the alkylating agent dacarbazine. Additional examples of chemotherapeutics agents include, e.g., paclitaxel or topotecan or pegylated liposomal doxorubicin (PLD). Other examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin; bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gamma1I and calicheamicin omegaI1 (see, e.g., Agnew, Chem. Intl. Ed. Engl., 33: 183-186 (1994)); dynemicin, including dynemicin A; bisphosphonates, such as clodronate; an esperamicin; as well as neocarzinostatin chromophore and related chromoprotein enediyne antiobiotic chromophores), aclacinomysins, actinomycin, authramycin, azaserine, bleomycins, cactinomycin, carabicin, caminomycin, carzinophilin, chromomycinis, dactinomycin, daunorubicin, detorubicin, 6-diazo-5-oxo-L-norleucine, ADRIAMYCIN® doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK® polysaccharide complex (JHS Natural Products, Eugene, Oreg.); razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g., TAXOL® paclitaxel (Bristol-Myers Squibb Oncology, Princeton, N.J.), ABRAXANE® Cremophor-free, albumin-engineered nanoparticle formulation of paclitaxel (American Pharmaceutical Partners, Schaumberg, Ill.), and TAXOTERE® docetaxel (Rhone-Poulenc Rorer, Antony, France); chloranbucil; GEMZAR® gemcitabine; 6-thioguanine; mercaptopurine; methotrexate; platinum analogs such as cisplatin, oxaliplatin and carboplatin; vinblastine; platinum; etoposide (VP-16); ifosfamide; mitoxantrone; vincristine; NAVELBINE® vinorelbine; novantrone; teniposide; edatrexate; daunomycin; aminopterin; xeloda; ibandronate; irinotecan (Camptosar, CPT-11) (including the treatment regimen of irinotecan with 5-FU and leucovorin); topoisomerase inhibitor RFS 2000; difluoromethylornithine (DMFO); retinoids such as retinoic acid; capecitabine; combretastatin; leucovorin (LV); oxaliplatin, including the oxaliplatin treatment regimen (FOLFOX); lapatinib (Tykerb®); inhibitors of PKC-alpha, Raf, H-Ras, EGFR (e.g., erlotinib (Tarceva®)) and VEGF-A that reduce cell proliferation and pharmaceutically acceptable salts, acids or derivatives of any of the above.

The term "concurrently" is used herein to refer to administration of two or more therapeutic agents, where at least part of the administration overlaps in time. Accordingly, concurrent administration includes a dosing regimen when the administration of one or more agent(s) continues after discontinuing the administration of one or more other agent(s).

The term "effective amount" refers to an amount of a drug effective to treat a disease or disorder in a mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, for example, be measured by assessing the duration of survival, duration of progression free survival (PFS), the response rates (RR), duration of response, and/or quality of life.

The "epitope A4.6.1" refers to the epitope recognized by the anti-VEGF antibody bevacizumab (AVASTIN®) (see Muller Y et al., Structure 15 September 1998, 6:1153-1167). In certain embodiments of the invention, the anti-VEGF antibodies include, but are not limited to, a monoclonal antibody that binds to the same epitope as the monoclonal anti-VEGF antibody A4.6.1 produced by hybridoma ATCC HB 10709; a recombinant humanized anti-VEGF monoclonal antibody generated according to Presta et al. (1997) Cancer Res. 57:4593-4599.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR," as used herein, refers to each of the regions of an antibody variable domain which are hypervariable in sequence and/or form structurally defined loops ("hypervariable loops"). Generally, native four-chain antibodies comprise six HVRs; three in the VH (HI, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (HI), 53-55 (H2), and 96-101 (H3). (Chothia and Lesk, J. Mol. Biol. 196:901-917 (1987).) Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991).) With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-Ll, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (See Almagro and Fransson, Front. Biosci. 13:1619-1633 (2008).) Unless otherwise indicated, HVR residues and other residues in the variable domain (e.g., FR residues) are numbered herein according to Kabat et al., supra.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

For the methods of the present disclosure, the term "instructing" a subject means providing directions for applicable therapy, medication, treatment, treatment regimens, and the like, by any means, but preferably in writing, such as in the form of package inserts or other written promotional material.

The term "intravenous infusion" refers to introduction of a drug into the vein of an animal or human subject over a period of time greater than approximately 5 minutes, preferably between approximately 30 to 90 minutes, although, according to the invention, intravenous infusion is alternatively administered for 10 hours or less.

The term "intravenous bolus" or "intravenous push" refers to drug administration into a vein of an animal or human such that the body receives the drug in approximately 15 minutes or less, preferably 5 minutes or less.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

A "maintenance" dose herein refers to one or more doses of a therapeutic agent administered to the subject over or after a treatment period. Usually, the maintenance doses are administered at spaced treatment intervals, such as approximately every week, approximately every 2 weeks, approximately every 3 weeks, or approximately every 4 weeks.

By "maintenance therapy" is meant a therapeutic regimen that is given to reduce the likelihood of disease recurrence or progression. Maintenance therapy can be provided for any length of time, including extended time periods up to the life-span of the subject. Maintenance therapy can be provided after initial therapy or in conjunction with initial or additional therapies. Dosages used for maintenance therapy can vary and can include diminished dosages as compared to dosages used for other types of therapy. See also "maintenance" herein.

The term "marketing" is used herein to describe the promotion, selling or distribution of a product (e.g., drug). Marketing specifically includes packaging, advertising, and any business activity with the purpose of commercializing a product.

By "metastasis" or "metastatic" is meant the spread of cancer from its primary site to other places in the body. Cancer cells can break away from a primary tumor, penetrate into lymphatic and blood vessels, circulate through the bloodstream, and grow in a distant focus (metastasize) in normal tissues elsewhere in the body. Metastasis can be local or distant. Metastasis is a sequential process, contingent on tumor cells breaking off from the primary tumor, traveling through the bloodstream, and stopping at a distant site. At the new site, the cells establish a blood supply and can grow to form a life-threatening mass. Both stimulatory and inhibitory molecular pathways within the tumor cell regulate this behavior, and interactions between the tumor cell and host cells in the distant site are also significant.

By "monotherapy" is meant a therapeutic regimen that includes only a single therapeutic agent for the treatment of the cancer or tumor during the course of the treatment period. Monotherapy using a VEGF-specific antagonist means that the VEGF-specific antagonist is administered in the absence of an additional anti-cancer therapy during treatment period.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

For the methods of the present disclosure, the term "promoting" means offering, advertising, selling, or describing a particular drug, combination of drugs, or treatment modality, by any means, including writing, such as in the form of package inserts. Promoting herein refers to promotion of a therapeutic agent, such as a VEGF antagonist, e.g., anti-VEGF antibody or chemotherapeutic agent, for an indication, such as breast cancer treatment, where such promoting is authorized by the Food and Drug Administration (FDA) as having been demonstrated to be associated with statistically significant therapeutic efficacy and acceptable safety in a population of subjects.

"Progression free survival (PFS)" refers to the time from treatment (or randomization) to first disease progression or death. For example it is the time that the subject remains alive, without return of the cancer, e.g., for a defined period of time such as about 1 month, about 2 months, about 3 months, about 4, months, about 5 months, about 6 months, about 7 months, about 8 months, about 9 months, about 1 year, about 2 years, about 3 years, etc., from initiation of treatment or from initial diagnosis. In one aspect of the invention, PFS can be assessed by the MacDonald Response Criteria as described in MacDonald et al., J Clin Oncol 1990;8:1277-80).

A "population" of subjects refers to a group of subjects with cancer, such as in a clinical trial, or as seen by oncologists following FDA approval for a particular indication, such as breast cancer therapy.

By "subject" is meant a mammal, including, but not limited to, a human or non-human mammal, such as a bovine, equine, canine, ovine, or feline. Preferably, the subject is a human. Patients are also subjects herein.

"Survival" refers to the subject remaining alive, and includes progression free survival (PFS) and overall survival (OS). Survival can be estimated by the Kaplan-Meier method, and any differences in survival are computed using the stratified log-rank test.

"Overall survival" refers to the subject remaining alive for a defined period of time, such as about 1 year, about 2 years, about 3 years, about 4 years, about 5 years, about 10 years, etc., from initiation of treatment or from initial diagnosis. In the studies underlying the present invention the event used for survival analysis was death from any cause.

"Overall response rate" or "Objective response rate" (ORR) - the percentage of people who experience a decrease in the size (or amount for blood cancers) of the cancer for a minimum amount of time; ORR is the sum of the complete and partial response rates.

By "extending survival" or "increasing the likelihood of survival" is meant increasing PFS and/or OS in a treated subject relative to an untreated subject (i.e. relative to a subject not treated with a VEGF antibody), or relative to a control treatment protocol, such as treatment only with the chemotherapeutic agent, such as those uses in the standard of care for glioblastoma, such as, for example, temozolomide with or without radiotherapy. Survival is monitored for at least about one month, about two months, about four months, about six months, about nine months, or at least about 1 year, or at least about 2 years, or at least about 3 years, or at least about 4 years, or at least about 5 years, or at least about 10 years, etc., following the initiation of treatment or following the initial diagnosis.

Hazard ratio (HR) is a statistical definition for rates of events. For the purpose of the invention, hazard ratio is defined as representing the probability of an event in the experimental arm divided by the probability of an event in the control arm at any specific point in time. "Hazard ratio" in progression free survival analysis is a summary of the difference between two progression free survival curves, representing the reduction in the risk of death on treatment compared to control, over a period of follow-up.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "VEGF" or "VEGF-A" is used to refer to the 165-amino acid human vascular endothelial cell growth factor and related 121-, 145-, 189-, and 206-amino acid human vascular endothelial cell growth factors, as described by, e.g., Leung et al. Science, 246:1306 (1989), and Houck et al. Mol. Endocrin., 5:1806 (1991), together with the naturally occurring allelic and processed forms thereof. VEGF-A is part of a gene family including VEGF-B, VEGF-C, VEGF-D, VEGF-E, VEGF-F, and PlGF. VEGF-A primarily binds to two high affinity receptor tyrosine kinases, VEGFR-1 (Flt-1) and VEGFR-2 (Flk-1/KDR), the latter being the major transmitter of vascular endothelial cell mitogenic signals of VEGF-A. Additionally, neuropilin-1 has been identified as a receptor for heparin-binding VEGF-A isoforms, and may play a role in vascular development. The term "VEGF" or "VEGF-A" also refers to VEGFs from non-human species such as mouse, rat, or primate. Sometimes the VEGF from a specific species is indicated by terms such as hVEGF for human VEGF or mVEGF for murine VEGF. Typically, VEGF refers to human VEGF. The term "VEGF" is also used to refer to truncated forms or fragments of the polypeptide comprising amino acids 8 to 109 or 1 to 109 of the 165-amino acid human vascular endothelial cell growth factor. Reference to any such forms of VEGF may be identified in the application, e.g., by "VEGF (8-109)," "VEGF (1-109)" or "VEGF165." The amino acid positions for a "truncated" native VEGF are numbered as indicated in the native VEGF sequence. For example, amino acid position 17 (methionine) in truncated native VEGF is also position 17 (methionine) in native VEGF. The truncated native VEGF has binding affinity for the KDR and Flt-1 receptors comparable to native VEGF.

An "anti-VEGF antibody" is an antibody that binds to VEGF with sufficient affinity and specificity. The antibody selected will normally have a binding affinity for VEGF, for example, the antibody may bind hVEGF with a Kd value of between 100 nM-1 pM. Antibody affinities may be determined by a surface plasmon resonance based assay (such as the BIAcore assay as described in PCT Application Publication No. WO2005/012359); enzyme-linked immunoabsorbent assay (ELISA); and competition assays (e.g. RIA's), for example. In certain embodiments, the anti-VEGF antibody of the invention can be used as a therapeutic agent in targeting and interfering with diseases or conditions wherein the VEGF activity is involved. Also, the antibody may be subjected to other biological activity assays, e.g., in order to evaluate its effectiveness as a therapeutic. Such assays are known in the art and depend on the target antigen and intended use for the antibody. Examples include the HUVEC inhibition assay; tumor cell growth inhibition assays (as described in WO 89/06692, for example); antibody-dependent cellular cytotoxicity (ADCC) and complement-mediated cytotoxicity (CDC) assays (U.S. Pat. No. 5,500,362); and agonistic activity or hematopoiesis assays (see WO 95/27062). An anti-VEGF antibody will usually not bind to other VEGF homologues such as VEGF-B or VEGF-C, nor other growth factors such as PlGF, PDGF or bFGF.

A "VEGF antagonist" refers to a molecule capable of neutralizing, blocking, inhibiting, abrogating, reducing or interfering with VEGF activities including its binding to one or more VEGF receptors. VEGF antagonists include anti-VEGF antibodies and antigen-binding fragments thereof, receptor molecules and derivatives which bind specifically to VEGF thereby sequestering its binding to one or more receptors, anti-VEGF receptor antibodies and VEGF receptor antagonists such as small molecule inhibitors of the VEGFR tyrosine kinases.

A "chimeric VEGF receptor protein" is a VEGF receptor molecule having amino acid sequences derived from at least two different proteins, at least one of which is a VEGF receptor protein. In certain embodiments, the chimeric VEGF receptor protein is capable of binding to and inhibiting the biological activity of VEGF.

### Chimeric and Humanized Antibodies

In certain embodiments, an antibody provided herein is a chimeric antibody. Certain chimeric antibodies are described, e.g., in U.S. Patent No. 4,816,567; and Morrison et al., PNAS USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. PNAS USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### Human Antibodies

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., PNAS USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### Library-Derived Antibodies

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, PNAS USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement in vitro, as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### Anti-VEGF Antibodies and Antagonists

The VEGF antigen to be used for production of VEGF antibodies may be, e.g., the VEGF₁₆₅ molecule as well as other isoforms of VEGF or a fragment thereof containing the desired epitope. In one embodiment, the desired epitope is the one recognized by bevacizumab, which binds to the same epitope as the monoclonal anti-VEGF antibody A4.6.1 produced by hybridoma ATCC HB 10709 (known as "epitope A.4.6.1" defined herein). Other forms of VEGF useful for generating anti-VEGF antibodies of the invention will be apparent to those skilled in the art.

Human VEGF was obtained by first screening a cDNA library prepared from human cells, using bovine VEGF cDNA as a hybridization probe. Leung et al. (1989) Science, 246:1306. One cDNA identified thereby encodes a 165-amino acid protein having greater than 95% homology to bovine VEGF; this 165-amino acid protein is typically referred to as human VEGF (hVEGF) or VEGF₁₆₅. The mitogenic activity of human VEGF was confirmed by expressing the human VEGF cDNA in mammalian host cells. Media conditioned by cells transfected with the human VEGF cDNA promoted the proliferation of capillary endothelial cells, whereas control cells did not. Leung et al. (1989) Science, supra. Further efforts were undertaken to clone and express VEGF via recombinant DNA techniques. (See, e.g., Ferrara, Laboratory Investigation 72:615-618 (1995), and the references cited therein).

VEGF is expressed in a variety of tissues as multiple homodimeric forms (121, 145, 165, 189, and 206 amino acids per monomer) resulting from alternative RNA splicing. VEGF₁₂₁ is a soluble mitogen that does not bind heparin; the longer forms of VEGF bind heparin with progressively higher affinity. The heparin-binding forms of VEGF can be cleaved in the carboxy terminus by plasmin to release a diffusible form(s) of VEGF. Amino acid sequencing of the carboxy terminal peptide identified after plasmin cleavage is Arg₁₁₀-Ala₁₁₁. Amino terminal "core" protein, VEGF (1-110) isolated as a homodimer, binds neutralizing monoclonal antibodies (such as the antibodies referred to as 4.6.1 and 3.2E3.1.1) and soluble forms of VEGF receptors with similar affinity compared to the intact VEGF₁₆₅ homodimer.

Several molecules structurally related to VEGF have also been identified recently, including placenta growth factor (PIGF), VEGF-B, VEGF-C, VEGF-D and VEGF-E. Ferrara and Davis-Smyth (1987) Endocr. Rev., supra; Ogawa et al. J. Biological Chem. 273:31273-31281 (1998); Meyer et al. EMBO J., 18:363-374 (1999). A receptor tyrosine kinase, Flt-4 (VEGFR-3), has been identified as the receptor for VEGF-C and VEGF-D. Joukov et al. EMBO. J. 15:1751 (1996); Lee et al. PNAS USA 93:1988-1992 (1996); Achen et al. (1998) PNAS USA 95:548-553. VEGF-C has been shown to be involved in the regulation of lymphatic angiogenesis. Jeltsch et al. Science 276:1423-1425 (1997).

Two VEGF receptors have been identified, Flt-1 (also called VEGFR-1) and KDR (also called VEGFR-2). Shibuya et al. (1990) Oncogene 8:519-527; de Vries et al. (1992) Science 255:989-991; Terman et al. (1992) Biochem. Biophys. Res. Commun. 187:1579-1586. Neuropilin-1 has been shown to be a selective VEGF receptor, able to bind the heparin-binding VEGF isoforms (Soker et al. (1998) Cell 92:735-45).

Anti-VEGF antibodies that are for use in accordance with the invention include any antibody, or antigen binding fragment thereof, that bind with sufficient affinity and specificity to VEGF and can reduce or inhibit the biological activity of VEGF. An anti-VEGF antibody will usually not bind to other VEGF homologues such as VEGF-B or VEGF-C, nor other growth factors such as PlGF, PDGF, or bFGF.

In certain embodiments of the invention, the anti-VEGF antibodies include, but are not limited to, a monoclonal antibody that binds to the same epitope as the monoclonal anti-VEGF antibody A4.6.1 produced by hybridoma ATCC HB 10709; a recombinant humanized anti-VEGF monoclonal antibody generated according to Presta et al. (1997) Cancer Res. 57:4593-4599. In one embodiment, the anti-VEGF antibody is "bevacizumab (BV)", also known as "rhuMAb VEGF" or "AVASTIN®". It comprises mutated human IgG1 framework regions and antigen-binding complementarity-determining regions from the murine anti-hVEGF monoclonal antibody A.4.6.1 that blocks binding of human VEGF to its receptors. Approximately 93% of the amino acid sequence of bevacizumab, including most of the framework regions, is derived from human IgG1, and about 7% of the sequence is derived from the murine antibody A4.6.1.

Bevacizumab (AVASTIN®) was the first anti-angiogenesis therapy approved by the FDA and is approved for the treatment metastatic colorectal cancer (first- and second-line treatment in combination with intravenous 5-FU-based chemotherapy), advanced non-squamous, non-small cell lung cancer (NSCLC) (first-line treatment of unresectable, locally advanced, recurrent or metastatic NSCLC in combination with carboplatin and paclitaxel) and metastatic HER2-negative breast cancer (previously untreated, metastatic HER2-negative breast cancer in combination with paclitaxel).

Bevacizumab and other humanized anti-VEGF antibodies are further described in U.S. Pat. No. 6,884,879 issued Feb. 26, 2005. Additional antibodies include the G6 or B20 series antibodies (e.g., G6-31, B20-4.1), as described in PCT Publication No. WO2005/012359, PCT Publication No. WO2005/044853, and U.S. Patent Application 60/991,302. For additional antibodies see U.S. Pat. Nos. 7,060,269, 6,582,959, 6,703,020; 6,054,297; WO98/45332; WO 96/30046; WO94/10202; EP 0666868B1; U.S. Patent Application Publication Nos. 2006009360, 20050186208, 20030206899, 20030190317, 20030203409, and 20050112126; and Popkov et al., Journal of Immunological Methods 288:149-164 (2004). Other antibodies include those that bind to a functional epitope on human VEGF comprising of residues F17, M18, D19, Y21, Y25, Q89, I191, K101, E103, and C104 or, alternatively, comprising residues F17, Y21, Q22, Y25, D63, I83 and Q89.

In one embodiment of the invention, the anti-VEGF antibody has a light chain variable region comprising the following amino acid sequence:
DIQMTQSPSS LSASVGDRVT ITCSASQDIS NYLNWYQQKP GKAPKVLIYF TSSLHSGVPS RFSGSGSGTD FTLTISSLQP EDFATYYCQQ YSTVPWTFGQ GTKVEIKR. (SEQ ID NO:1)

and a heavy chain variable region comprising the following amino acid sequence:
EVQLVESGGG LVQPGGSLRL SCAASGYTFT NYGMNWVRQA PGKGLEWVGW INTYTGEPTY AADFKRRFTF SLDTSKSTAY LQMNSLRAED TAVYYCAKYP HYYGSSHWYF DVWGQGTLVT VSS (SEQ ID NO:2)

A "G6 series antibody" according to this invention, is an anti-VEGF antibody that is derived from a sequence of a G6 antibody or G6-derived antibody according to anyone of FIGS. 7, 24-26, and 34-35 of PCT Publication No. WO2005/012359,. See also PCT Publication No. WO2005/044853. In one embodiment, the G6 series antibody binds to a functional epitope on human VEGF comprising residues F17, Y21, Q22, Y25, D63, 183 and Q89.

A "B20 series antibody" according to this invention is an anti-VEGF antibody that is derived from a sequence of the B20 antibody or a B20-derived antibody according to any one of FIGS. 27-29 of PCT Publication No. WO2005/012359. See also PCT Publication No. WO2005/044853, and U.S. Patent Application 60/991,302. In one embodiment, the B20 series antibody binds to a functional epitope on human VEGF comprising residues F17, M18, D19, Y21, Y25, Q89, I91, K101, E103, and C104.

A "functional epitope" according to this invention refers to amino acid residues of an antigen that contribute energetically to the binding of an antibody. Mutation of any one of the energetically contributing residues of the antigen (for example, mutation of wild-type VEGF by alanine or homolog mutation) will disrupt the binding of the antibody such that the relative affinity ratio (IC50mutant VEGF/IC50wild-type VEGF) of the antibody will be greater than 5 (see Example 2 of WO2005/012359). In one embodiment, the relative affinity ratio is determined by a solution binding phage displaying ELISA. Briefly, 96-well Maxisorp immunoplates (NUNC) are coated overnight at 4°C with an Fab form of the antibody to be tested at a concentration of 2 µg/ml in PBS, and blocked with PBS, 0.5% BSA, and 0.05% Tween20 (PBT) for 2 h at room temperature. Serial dilutions of phage displaying hVEGF alanine point mutants (residues 8-109 form) or wild type hVEGF (8-109) in PBT are first incubated on the Fab-coated plates for 15 min at room temperature, and the plates are washed with PBS, 0.05% Tween20 (PBST). The bound phage is detected with an anti-M13 monoclonal antibody horseradish peroxidase (Amersham Pharmacia) conjugate diluted 1:5000 in PBT, developed with 3,3',5,5'-tetramethylbenzidine (TMB, Kirkegaard & Perry Labs, Gaithersburg, Md.) substrate for approximately 5 min, quenched with 1.0 M H3PO4, and read spectrophotometrically at 450 nm. The ratio of IC50 values (IC50,ala/IC50,wt) represents the fold of reduction in binding affinity (the relative binding affinity).

### VEGF Receptor Molecules

The two best characterized VEGF receptors are VEGFR1 (also known as Flt-1) and VEGFR2 (also known as KDR and FLK-1 for the murine homolog). The specificity of each receptor for each VEGF family member varies but VEGF-A binds to both Flt-1 and KDR. Both Flt-I and KDR belong to the family of receptor tyrosine kinases (RTKs). The RTKs comprise a large family of transmembrane receptors with diverse biological activities. At least nineteen (19) distinct RTK subfamilies have been identified. The receptor tyrosine kinase (RTK) family includes receptors that are crucial for the growth and differentiation of a variety of cell types (Yarden and Ullrich (1988) Ann. Rev. Biochem. 57:433-478; Ullrich and Schlessinger (1990) Cell 61:243-254). The intrinsic function of RTKs is activated upon ligand binding, which results in phosphorylation of the receptor and multiple cellular substrates, and subsequently in a variety of cellular responses (Ullrich & Schlessinger (1990) Cell 61:203-212). Thus, receptor tyrosine kinase mediated signal transduction is initiated by extracellular interaction with a specific growth factor (ligand), typically followed by receptor dimerization, stimulation of the intrinsic protein tyrosine kinase activity and receptor trans-phosphorylation. Binding sites are thereby created for intracellular signal transduction molecules and lead to the formation of complexes with a spectrum of cytoplasmic signaling molecules that facilitate the appropriate cellular response. (e.g., cell division, differentiation, metabolic effects, changes in the extracellular microenvironment) see, Schlessinger and Ullrich (1992) Neuron 9:1-20. Structurally, both Flt-1 and KDR have seven immunoglobulin-like domains in the extracellular domain, a single transmembrane region, and a consensus tyrosine kinase sequence which is interrupted by a kinase-insert domain. Matthews et al. (1991) PNAS USA 88:9026-9030; Terman et al. (1991) Oncogene 6:1677-1683. The extracellular domain is involved in the binding of VEGF and the intracellular domain is involved in signal transduction.

VEGF receptor molecules, or fragments thereof, that specifically bind to VEGF can be used to bind to and sequester the VEGF protein, thereby preventing it from signaling. In certain embodiments, the VEGF receptor molecule, or VEGF binding fragment thereof, is a soluble form, such as sFlt-1. A soluble form of the receptor exerts an inhibitory effect on the biological activity of the VEGF protein by binding to VEGF, thereby preventing it from binding to its natural receptors present on the surface of target cells. Also included are VEGF receptor fusion proteins, examples of which are described below.

A chimeric VEGF receptor protein is a receptor molecule having amino acid sequences derived from at least two different proteins, at least one of which is a VEGF receptor protein (e.g., the flt-1 or KDR receptor), that is capable of binding to and inhibiting the biological activity of VEGF. In certain embodiments, the chimeric VEGF receptor proteins consist of amino acid sequences derived from only two different VEGF receptor molecules; however, amino acid sequences comprising one, two, three, four, five, six, or all seven Ig-like domains from the extracellular ligand-binding region of the flt-1 and/or KDR receptor can be linked to amino acid sequences from other unrelated proteins, for example, immunoglobulin sequences. Other amino acid sequences to which Ig-like domains are combined will be readily apparent to those of ordinary skill in the art. Examples of chimeric VEGF receptor proteins include, e.g., soluble Flt-1/Fc, KDR/Fc, or FLt-1/KDR/Fc (also known as VEGF Trap). (See for example PCT Application Publication No. WO97/44453).

A soluble VEGF receptor protein or chimeric VEGF receptor proteins includes VEGF receptor proteins which are not fixed to the surface of cells via a transmembrane domain. As such, soluble forms of the VEGF receptor, including chimeric receptor proteins, while capable of binding to and inactivating VEGF, do not comprise a transmembrane domain and thus generally do not become associated with the cell membrane of cells in which the molecule is expressed.

### Therapeutic Uses and Compositions

The invention encompasses anti-angiogenic therapy, a novel cancer treatment strategy aimed at inhibiting the development of tumor blood vessels required for providing nutrients to support tumor growth. Because angiogenesis is involved in both primary tumor growth and metastasis, the anti-angiogenic treatment provided by the invention is capable of inhibiting the neoplastic growth of tumor at the primary site as well as preventing metastasis of tumors at the secondary sites, therefore allowing attack of the tumors by other therapeutics.

Specifically, provided herein are means for treating a subject diagnosed with glioblastoma, by administering to the subject a treatment regimen combining an effective amount of a chemotherapeutic and an anti-VEGF antibody. The treatment regimen combining the chemotherapy and the administration of the anti-VEGF antibody extends the progression free survival (PFS) or the overall survival (OS) of the subject.

### Combination Therapies

The invention features the use or compositions of a combination of an anti-VEGF antibody with one or more additional anti-cancer therapies. Examples of anti-cancer therapies include, without limitation, surgery, radiation therapy (radiotherapy), biotherapy, immunotherapy, chemotherapy (e.g., temozolomide), or a combination of these therapies. In addition, cytotoxic agents, anti-angiogenic and anti-proliferative agents can be used in combination with the anti-VEGF antibody.

In certain aspects of any of the methods and uses, the invention provides treating glioblastoma, by administering effective amounts of an anti-VEGF antibody and a chemotherapeutic agents to a subject diagnosed with glioblastoma. A variety of chemotherapeutic agents may be used in the combined treatment methods and uses of the invention. An exemplary and non-limiting list of chemotherapeutic agents contemplated is provided herein under "Definition", or described herein. In one embodiment, the chemotherapeutic agent is temolozolomide. In another embodiment, the chemotherapeutic agent is administered concommitantly with radiotherapy.

In one example, the combined treatment contemplated above involves administration which includes simultaneous administration, using separate formulations or a single pharmaceutical formulation, and consecutive administration in either order, wherein preferably there is a time period while both (or all) active agents simultaneously exert their biological activities. Preparation and dosing schedules for such chemotherapeutic agents may be used according to manufacturers' instructions or as determined empirically by the skilled practitioner. Preparation and dosing schedules for chemotherapy are also described in Chemotherapy Service Ed., M. C. Perry, Williams & Wilkins, Baltimore, Md. (1992). The chemotherapeutic agent may precede, or follow administration of the anti-VEGF antibody or may be given simultaneously therewith.

In some other aspects of any of the methods and uses, other therapeutic agents useful for combination tumor therapy with the antibody of the invention include antagonist of other factors that are involved in tumor growth, such as EGFR, ErbB3, ErbB4, or TNF. Sometimes, it may be beneficial to also administer one or more cytokines to the subject. In one embodiment, the VEGF antibody is co-administered with a growth inhibitory agent. For example, the growth inhibitory agent may be administered first, followed by the VEGF antibody. However, simultaneous administration or administration of the VEGF antibody first is also contemplated. Suitable dosages for the growth inhibitory agent are those presently used and may be lowered due to the combined action (synergy) of the growth inhibitory agent and anti-VEGF antibody.

The formulation herein may also contain more than one active compound as necessary for the particular indication being treated, preferably those with complementary activities that do not adversely affect each other. For example, it may be desirable to further provide antibodies which bind to EGFR, VEGF (e.g. an antibody which binds a different epitope or same epitope on VEGF), VEGFR, or ErbB2 (e.g., Herceptin®) in the one formulation. Alternatively, or in addition, the composition may comprise a chemotherapeutic agent, or a cytotoxic agent. Such molecules are suitably present in combination in amounts that are effective for the purpose intended.

In certain aspects of any of the methods and uses, other therapeutic agents useful for combination cancer therapy with the antibody of the invention include other anti-angiogenic agents. Many anti-angiogenic agents have been identified and are known in the arts, including those listed by Carmeliet and Jain (2000). In one embodiment, the anti-VEGF antibody of the invention is used in combination with another VEGF antagonist or a VEGF receptor antagonist such as VEGF variants, soluble VEGF receptor fragments, aptamers capable of blocking VEGF or VEGFR, neutralizing anti-VEGFR antibodies, low molecule weight inhibitors of VEGFR tyrosine kinases and any combinations thereof. Alternatively, or in addition, two or more anti-VEGF antibodies may be co-administered to the subject.

For the prevention or treatment of disease, the appropriate dosage of VEGF-specific antagonist will depend on the type of disease to be treated, as defined above, the severity and course of the disease, whether the VEGF-specific antagonist is administered for preventive or therapeutic purposes, previous therapy, the subject's clinical history and response to the VEGF-specific antagonist, and the discretion of the attending physician. The VEGF-specific antagonist is suitably administered to the subject at one time or over a series of treatments. In a combination therapy regimen, the VEGF-specific antagonist and the one or more anti-cancer therapeutic agent of the invention are administered in a therapeutically effective or synergistic amount. As used herein, a therapeutically effective amount is such that co-administration of a VEGF-specific antagonist and one or more other therapeutic agents, or administration of a composition of the invention, results in reduction or inhibition of the cancer as described above. A therapeutically synergistic amount is that amount of a VEGF-specific antagonist and one or more other therapeutic agents necessary to synergistically or significantly reduce or eliminate conditions or symptoms associated with a particular disease.

The VEGF-specific antagonist and the one or more other therapeutic agents can be administered simultaneously or sequentially in an amount and for a time sufficient to reduce or eliminate the occurrence or recurrence of a tumor, a dormant tumor, or a micrometastases. The VEGF-specific antagonist and the one or more other therapeutic agents can be administered as maintenance therapy to prevent or reduce the likelihood of recurrence of the tumor.

As will be understood by those of ordinary skill in the art, the appropriate doses of chemotherapeutic agents or other anti-cancer agents will be generally around those already employed in clinical therapies, e.g., where the chemotherapeutics are administered alone or in combination with other chemotherapeutics. Variation in dosage will likely occur depending on the condition being treated. The physician administering treatment will be able to determine the appropriate dose for the individual subject.

In addition to the above therapeutic regimes, the subject may be subjected to radiation therapy.

In certain embodiments of any of the methods, uses and compositions, the administered VEGF antibody antibody is an intact, naked antibody. However, the VEGF antibody may be conjugated with a cytotoxic agent. In certain embodiments of any of the methods and uses, the conjugated antibody and/or antigen to which it is bound is/are internalized by the cell, resulting in increased therapeutic efficacy of the conjugate in killing the cancer cell to which it binds. In one embodiment, the cytotoxic agent targets or interferes with nucleic acid in the cancer cell. Examples of such cytotoxic agents include maytansinoids, calicheamicins, ribonucleases and DNA endonucleases.

The disclosure also features a method of instructing a human subject with glioblastoma or a health care provider by providing instructions to receive treatment with an anti-VEGF antibody in combination with a chemotherapeutic (e.g., temozolomide) and one or more other therapeutic agents e.g., radiotherapy) so as to increase the time for progression free survival, to decrease the subject's risk of cancer recurrence or to increase the subject's likelihood of survival. In some embodiments the method further comprises providing instructions to receive treatment with at least one chemotherapeutic agent. The treatment with the anti-VEGF antibody may be concurrent with or sequential to the treatment with the chemotherapeutic agent. In certain embodiments the subject is treated as instructed by the method of instructing. Treatment of glioblastoma by administration of an anti-VEGF antibody with or without chemotherapy with or without the other therapeutics agents may be continued until cancer recurrence or death.

The disclosure further provides a promotional method, comprising promoting the administration of an anti-VEGF antibody and one or more other therapeutic agents for treatment of glioblastoma in a human subject. In some embodiments the method further comprises promoting the administration of at least one chemotherapeutic agent. Administration of the anti-VEGF antibody may be concurrent with or sequential to administration of the chemotherapeutic agent. Promotion may be conducted by any means available. In some embodiments the promotion is by a package insert accompanying a commercial formulation of the anti-VEGF antibody. The promotion may also be by a package insert accompanying a commercial formulation of the chemotherapeutic agent. Promotion may be by written or oral communication to a physician or health care provider. In some embodiments the promotion is by a package insert where the package inset provides instructions to receive glioblastoma therapy with anti-VEGF antibody in combination with one or more other chemotherapeutics or therapeutic agents. In a further embodiment, the package insert include some or all of the results under Example 1. In some embodiments the promotion is followed by the treatment of the subject with the anti-VEGF antibody with the chemotherapeutic agent and other therapeutic agent.

The disclosure provides a business method, comprising marketing an anti-VEGF antibody in combination with one or more other therapeutic agents for treatment of glioblastoma in a human subject so as to increase the subject's time for progression free survival, to decrease the subject's likelihood of cancer recurrence or increase the subject's likelihood of survival. In some embodiments the method further comprises marketing a chemotherapeutic agent for use in combination with the anti-VEGF antibody. In some embodiments the marketing is followed by treatment of the subject with the anti-VEGF antibody with the chemotherapeutic agent.

Also disclosed is a business method, comprising marketing a chemotherapeutic agent in combination with an anti-VEGF antibody for treatment of glioblastoma in a human subject so as to increase the subject's time for progression free survival, to decrease the subject's likelihood of cancer recurrence or increase the subject's likelihood of survival. In some embodiments, the marketing is followed by treatment of the subject with the combination of the chemotherapeutic agent and the anti-VEGF antibody.

### Dosages and Duration

The invention will be formulated, dosed, and administered in a fashion consistent with good medical practice. Factors for consideration in this context include the particular disorder being treated, the particular subject being treated, the clinical condition of the individual subject, the cause of the disorder, the site of delivery of the agent, the method of administration, the scheduling of administration, and other factors known to medical practitioners. The "therapeutically effective amount" of the invention to be administered will be governed by such considerations, and is the minimum amount necessary to prevent, ameliorate, or treat, or stabilize, the cancer; to increase the time until progression (duration of progression free survival) or to treat or prevent the occurrence or recurrence of a tumor, a dormant tumor, or a micrometastases. The VEGF-specific antagonist need not be, but is optionally, formulated with one or more agents currently used to prevent or treat cancer or a risk of developing a cancer. The effective amount of such other agents depends on the amount of VEGF-specific antagonist present in the formulation, the type of disorder or treatment, and other factors discussed above. These are generally used in the same dosages and with administration routes as used hereinbefore or about from 1 to 99% of the heretofore employed dosages.

Depending on the type and severity of the disease, about 1 ug/kg to 100 mg/kg (e.g., 0.1-20 mg/kg) of either the anti-VEGF antibody as an initial candidate dosage for administration to the subject, whether, for example, by one or more separate administrations, or by continuous infusion. In one embodiment, desirable dosages include, for example, 6 mg/kg, 8 mg/kg, 10 mg/kg, and 15 mg/kg. For repeated administrations or cycles over several days or longer, depending on the condition, the treatment is sustained until the cancer is treated, as measured by the methods described above or known in the art. However, other dosage regimens may be useful. In one example, the anti-VEGF antibody is administered once every week, every two weeks, or every three weeks, at a dose range from about 6 mg/kg to about 15 mg/kg, including but not limited to 6 mg/kg, 8 mg/kg, 10 mg/kg or 15 mg/kg. The progress of the therapy of the invention is easily monitored by conventional techniques and assays. In other embodiments, such dosing regimen is used in combination with a chemotherapy regimen in glioblastoma. Further information about suitable dosages is provided in the Example below.

The duration of therapy will continue for as long as medically indicated or until a desired therapeutic effect (e.g., those described herein) is achieved. In certain embodiments, the claimed therapy is continued for 1 month, 2 months, 4 months, 6 months, 8 months, 10 months, 1 year, 2 years, 3 years, 4 years, 5 years, or for a period of years up to the lifetime of the subject.

The VEGF-specific antagonists of the invention are administered to a subject, e.g., a human subject, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Local administration is particularly desired if extensive side effects or toxicity is associated with the VEGF antagonist. An ex vivo strategy can also be used for therapeutic applications. Ex vivo strategies involve transfecting or transducing cells obtained from the subject with a polynucleotide encoding a VEGF antagonist. The transfected or transduced cells are then returned to the subject. The cells can be any of a wide range of types including, without limitation, hematopoietic cells (e.g., bone marrow cells, macrophages, monocytes, dendritic cells, T cells, or B cells), fibroblasts, epithelial cells, endothelial cells, keratinocytes, or muscle cells.

For example, if the VEGF-specific antagonist is an antibody, the antibody is administered by any suitable means, including parenteral, subcutaneous, intraperitoneal, intrapulmonary, and intranasal, and, if desired for local immunosuppressive treatment, intralesional administration. Parenteral infusions include intramuscular, intravenous, intraarterial, intraperitoneal, or subcutaneous administration. In addition, the antibody is suitably administered by pulse infusion, particularly with declining doses of the antibody. Preferably the dosing is given by injections, most preferably intravenous or subcutaneous injections, depending in part on whether the administration is brief or chronic.

In another example, the VEGF antibody is administered locally, e.g., by direct injections, when the disorder or location of the tumor permits, and the injections can be repeated periodically. The VEGF antibody can also be delivered systemically to the subject or directly to the tumor cells, e.g., to a tumor or a tumor bed following surgical excision of the tumor, in order to prevent or reduce local recurrence or metastasis, for example of a dormant tumor or micrometastases.

### Pharmaceutical Formulations

Therapeutic formulations of the antibodies described herein, used in accordance with the invention, are prepared for storage by mixing an antibody having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG). Lyophilized anti-VEGF antibody formulations are described in WO 97/04801, expressly incorporated herein be reference.

Optionally, but preferably, the formulation contains a pharmaceutically acceptable salt, typically, e.g., sodium chloride, and preferably at about physiological concentrations. Optionally, the formulations of the invention can contain a pharmaceutically acceptable preservative. In some embodiments the preservative concentration ranges from 0.1 to 2.0%, typically v/v. Suitable preservatives include those known in the pharmaceutical arts. Benzyl alcohol, phenol, m-cresol, methylparaben, and propylparaben are examples of preservatives. Optionally, the formulations of the invention can include a pharmaceutically acceptable surfactant at a concentration of 0.005 to 0.02%.

Typically, bevacizumab is supplied for therapeutic uses in 100 mg and 400 mg preservative-free, single-use vials to deliver 4 ml or 16 ml of bevacizumab (25 mg/ml). The 100 mg product is formulated in 240 mg α, α-trehalose dehydrate, 23.2 mg sodium phosphate (monobasic, monohydrate), 4.8 mg sodium phosphate (dibasic, anhydrous), 1.6 mg polysorbate 20, and Water for Injection, USP. The 400 mg product is formulated in 960 mg α, α-trehalose dehydrate, 92.8 mg sodium phosphate (monobasic, monohydrate), 19.2 mg sodium phosphate (dibasic, anhydrous), 6.4 mg polysorbate 20, and Water for Injection, USP. See also the label for bevacizumab.

The formulation herein may also contain more than one active compound as necessary for the particular indication being treated, preferably those with complementary activities that do not adversely affect each other. For example, it may be desirable to further provide antibodies which bind to VEGF (e.g. an antibody which binds a different epitope on VEGF), VEGFR in the one formulation. Alternatively, or in addition, the composition may comprise a cytotoxic agent, cytokine, growth inhibitory agent and/or VEGFR antagonist. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and y ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C., resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

The formulations to be used for in vivo administration may be sterile. This is readily accomplished by filtration through sterile filtration membranes.

### Efficacy of the Treatment

The main advantage of the of any of the methods, uses and compositions provided herein is the ability of producing marked anti-cancer effects in a human subject without causing significant toxicities or adverse effects, so that the subject benefited from the treatment overall. In one embodiment of any of the methods, uses or compositions, the safety profile is comparable to previous bevacizumab phase III studies. The efficacy of the treatment of the invention can be measured by various endpoints commonly used in evaluating cancer treatments, including but not limited to, tumor regression, tumor weight or size shrinkage, time to progression, duration of survival, progression free survival, overall response rate, duration of response, and quality of life.

### Kits

In another embodiment of the disclosure, an article of manufacture containing materials useful for the treatment of the disorders described above is provided. The article of manufacture comprises a container, a label and a package insert. Suitable containers include, for example, bottles, vials, syringes, etc. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). At least one active agent in the composition is an anti-VEGF antibody. The label on, or associated with, the container indicates that the composition is used for treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, and syringes. In addition, the article of manufacture comprises a package inserts with instructions for use, including for example instructing the user of the composition to administer the anti-VEGF antibody composition and a chemotherapeutic agent to the subject, e.g., temozolomide. The package insert may optionally contain some or all of the results found in Example 1.

The anti-VEGF antibody can be packaged alone or in combination with other anti-cancer therapeutic compounds as a kit. The kit can include optional components that aid in the administration of the unit dose to subjects, such as vials for reconstituting powder forms, syringes for injection, customized IV delivery systems, inhalers, etc. Additionally, the unit dose kit can contain instructions for preparation and administration of the compositions. In certain embodiments, the instructions comprises instructions for use, including for example instructing the user of the composition to administer the anti-VEGF antibody composition and a chemotherapeutic agent to the subject, e.g., temozolomide. The instructions may optionally contain some or all of the results found in Example 1. The kit may be manufactured as a single use unit dose for one subject, multiple uses for a particular subject (at a constant dose or in which the individual compounds may vary in potency as therapy progresses); or the kit may contain multiple doses suitable for administration to multiple subjects ("bulk packaging"). The kit components may be assembled in cartons, blister packs, bottles, tubes, and the like.

### EXAMPLE

The following are examples of methods and compositions of the invention. It is understood that various other embodiments may be practiced, given the general description provided above.

### Example 1 - A randomized, double blind, placebo controlled multicenter Phase III trial of bevacizumab, temozolomide and radiotherapy, followed by bevacizumab and temozolomide versus placebo, temozolomide and radiotherapy followed by placebo and temozolomide in patients with newly diagnosed glioblastoma (AVAglio)

The AVAglio trial evaluated the efficacy and safety of bevacizumab in combination temozolomide and radiotherapy for newly diagnosed glioblastoma. This study was designed as a prospective, randomized, double blind, placebo controlled Phase III evaluation of bevacizumab plus chemotherapy versus chemotherapy alone. To be eligible, patients must have newly diagnosed glioblastoma with a tissue diagnosis that has been established following either a surgical resection or biopsy. By adding bevacizumab to chemotherapy and radiotherapy, the AVAglio trial aimed to improve overall survival (OS) and progression-free survival (PFS) for this group of patients who have limited therapeutic options and face a particularly poor prognosis. The primary objective was to compare OS and PFS of patients randomised to temozolomide and radiotherapy only or to temozolomide and radiotherapy plus bevacizumab.

Study Design - This trial consisted of three phases (Concurrent, Maintenance, and Monotherapy) and two (2) treatment arms: temozolomide and radiotherapy (Arm 1) and temozolomide and radiotherapy plus bevacizumab (Arm 2). Patients were randomly assigned (1:1) to either arm, see Figure 1.

Arm 1 (chemotherapy and radiotherapy alone): Eligible patients received 2 Gy radiotherapy 5 days a week for 6 weeks and 75 mg/m² temozolomide (TMZ) oraly daily for 6 weeks from the first day to the last day of radiotherapy in combination with 10 mg/kg bevacizumab IV every 2 weeks. After a 4 week treatment break, eligible patients received 6 cycles of 150-200 mg/m² TMZ on days 1-5 of a every 4 weeks schedule in combination with 10 mg/kg placebo IV every 2 weeks.. TMZ was administered orally starting with a 150 mg/m² dose that could be escalated. Placebo monotherapy (15 mg/kg every 3 weeks) was then continued until disease progression.

Upon disease progression, patients were treated at the investigator's discretion.

Arm 2 (TMZ and radiotherapy plus bevacizumab): Eligible patients received 2 Gy radiotherapy 5 days a week for 6 weeks and 75 mg/m² TMZ orally daily for 6 weeks from the first day to the last day of radiotherapy in combination with 10 mg/kg bevaciumab IV every 2 weeks. After a 4 week treatment break, eligible patients received 6 cycles of 150-200 mg/m² TMZ on days 1-5 of a every 4 weeks schedule in combination with 10 mg/kg bevaciumab IV every 2 weeks. TMZ was administered orally starting with a 150 mg/m² dose that could be escalated. Bevaciazumab monotherapy (15 mg/kg every 3 weeks) was then continued until disease progression.

Upon disease progression, patients were treated at the investigator's discretion.

The initial bevacizumab infusion was over 90 minutes, with subsequent infusions over 60 minutes and then 30 minutes, as tolerated. Bevacizumab was administered on the day of the last day of radiotherapy and TMZ, i.e., the day before the start of the TMZ treatment break.

Analyses of PFS was based on tumor assessments MacDonald Response Criteria (modified WHO criteria) using MRI of the brain and a neurological evalution as described in Macdonald et al., Response criteria for phase II studies of supratentorial malignant glioma. J Clin Oncol 1990;8:1277-80.

Tumor assessments were performed at baseline, at the end of the 4 week treatment break, then then every 8 weeks.

### Study Population - Inclusion Criteria

Patients ≥18 years of age and with newly diagnosed supratentorial Glioblastoma (GBM) with a tissue diagnosis that has been established following either a surgical resection or biopsy. This includes treatment-naive -(chemotherapy and radiotherapy)- patients with prior diagnosis of a lower grade astrocytoma that has been upgraded to a histologically verified GBM. Patients must have WHO performance status ≤ 2.

### Study Population - Exclusion criteria

Evidence of recent hemorrhage on postoperative MRI of the brain. However, patients with clinically asymptomatic presence of hemosiderin, resolving hemorrhagic changes related to surgery, and presence of punctate hemorrhage in the tumor are permitted entry into the study. Previous centralized screening for MGMT status for enrollment into a clinical trial; any prior chemotherapy (including carmustine-containing wafers (Gliadel®) or immunotherapy (including vaccine therapy) for glioblastomas and low grade astrocytomas; any prior radiotherapy to the brain or prior radiotherapy resulting in a potential overlap in the radiation field; prior history of hypertensive crisis or hypertensive encephalopathy; history of ≥ grade 2 haemoptysis according to the NCI-CTC criteria within 1 month prior to randomization; evidence of bleeding diathesis or coagulopathy (in the absence of therapeutic anticoagulation); major surgical procedure, open biopsy, intracranial biopsy, ventriculoperitoneal shunt or significant traumatic injury within 28 days prior to randomization; core biopsy (excluding intracranial biopsy) or other minor surgical procedure within 7 days prior to randomization. Placement of a central vascular access device (CVAD) if performed within 2 days prior to bevacizumab/placebo administration; history of abdominal fistula or gastrointestinal perforation within 6 months prior to randomization history of intracranial abscess within 6 months prior to randomization; erious non-healing wound, active ulcer or untreated bone fracture. Pregnant or lactating females. Serum pregnancy test to be assessed within 7 days prior to study treatment start, or within 14 days (with a confirmatory urine pregnancy test within 7 days prior to study treatment start); fertile women and men (defined as <2 years after last menstruation and not surgically sterile) not using highly-effective, hormonal or non-hormonal means of contraception (i.e. intrauterine contraceptive device); history of stroke or transient ischemic attack (TIA) within ≤6 months prior to randomization; inadequately controlled hypertension (sustained systolic >150 mmHg and/or diastolic >100 mmHg) or significant vascular disease, including: aortic aneurism requiring surgical repair or recent peripheral arterial thrombosis) within ≤6 months prior to randomization. Myocardial infarction or unstable angina within ≤6 months prior to randomization or New York Heart Association (NYHA) grade II or greater congestive heart failure (CHF); known hypersensitivity to any of the study drugs or excipients.

### RESULTS:

Eligible patients had newly diagnosed glioblastoma (WHO performance status ≤ 2). After surgical resection, patients were randomized to concurrent therapy with temozolomide with radiation and placebo or temozolomide with radiation and bevacizumab followed by a 28 day treatment break, maintenance therapy with 10 mg/kg bevacizumab or placebo administered every two weeks in combination with temozolomide, and monotherapy with 15 mg/kg bevacizumab or placebo was administered every three weeks until disease progression or unacceptable toxicity.. Patients in the placebo arm were treated at the investigators discretion at progression. The design provided 80% power to detect a PFS hazard ratio (HR) of 0.769 with 2-sided log-rank test and α=0.01 after 677 events, assuming median PFS of 7.0 mo with temozolomide + radiotherapy + placebo and 9.1 mo with temozolomide + radiotherapy + bevacizumab.

Between June 2009 and March 2011, 921 patients were randomized to receive temozolomide with radiation and placebo or temozolomide with radiation and bevacizumab. Median follow-up was 13.7 months for placebo + temozolomide + radiotherapy and 14.4 months for bevacizumab + temozolomide + radiotherapy.

**TABLE 1: AVAglio PHASE III RESULTS**

| | Placebo (PL) + Radiotherapy (RT) + Temozolomide (T) (N=463) | bevacizumab + RT + T (n-458) |
|---|---|---|
| PFS (months) | 6.2 | 10.6 |
| | (N=182) | (N=179) |
| Events, n (%) | 387 (83.6) | 354 (77.3) |
| HR (95% CI) | 0.64 (0.55 - 0.74) | |
| | Log-rank p<0.0001 | |
| Hypertension (Grade ≥3) | 2 | 10.3 |
| Proteinuria (Grade ≥3) | 0 | 3.7 |

| Bleeding (all grade) | | |
|---|---|---|
| • Cerebral Hemorrhage | 2.2 | 2.6 |
| • Mucocutaneous bleeding | 8.9 | 26.7 |
| • Other | 8.1 | 11.6 |

| Bleeding (Grade ≥3) | | |
|---|---|---|
| • Cerebral Hemorrhage | 0.7 | 1.5 |
| • Mucocutaneous bleeding | 0 | 0.4 |
| • Other | 0.4 | 0.6 |

| Thromboembolic event | | |
|---|---|---|
| Arterial (all grade) | 1.6 | 5 |
| Venous (Grade ≥3) | 8.1 | 7.3 |
| GI perforation (all grade) | 0.2 | 1.7 |
| Fistula/abscess (all grade) | 0.4 | 0.6 |
| PRES (Posterior reversible encephalopathy syndrome) (all grade) | 0 | 0 |
| CHF (grade ≥ 3) | 0 | 0.4 |

Figure 2 shows the Kaplan Meier curves for PFS in AVAglio.

AVAglio is the first randomized trial of bevacizumab in newly diagnosed glioblastoma. As shown in Table 1 above, bevacizumab and chemotherapy in combination with radiotherapy provides statistically significant and clinically meaningful improvement in PFS versus chemotherapy in combination with radiotherapy. Careful patient screening minimizes the risk of bevacizumab adverse events. With bevacizumab, 66% of patients on steroids at baseline were able to discontinue steroids for part of their progression-free survival interval versus 47% of patients on placebo. As shown in Table 2 below, addition of bevacizumab to first line temozolomide plus radiotherapy significantly improves ORR.

**Table 2**

| | **Best ORR** | | |
|---|---|---|---|
| | Bv+T/RT | | P+T/RT |
| n | 375 | | 366 |
| Responders, n % (95% CI) | 144 38.4 (33.5-43.5) | | 66 18 (14.2-22.4) |
| Difference, % (95% CI) | | 20.4 (13.9-26.8) | |
| | | p=<0.001 | |

| | **PsPD** | | |
|---|---|---|---|
| n | 458 | | 463 |

| | *End of treatment break* | | |
|---|---|---|---|
| Potential PsPD, n (%) | 12 (2.6) | | 84 (18.1) |

| | *Post-2nd maintenance cycle* | | |
|---|---|---|---|
| Confirmed PsPD, n (%) | 10 (2.2) | | 43 (9.3) |
| Rejected PsPD, n (%) | 1 (0.2) | | 35 (7.6) |
| Missing, n (%) | 1 (0.2) | | 6 (1.3) |

This is the first phase III trial in glioblastoma to show benefit with a combination of bevacizumab, chemotherapy and radiotherapy and versus chemotherapy and radiotherapy.

## Claims

1. An anti-VEGF antibody for use in a method of treating a patient diagnosed with a glioblastoma, wherein the method comprises administering to said patient a therapy comprising an effective amount of an anti-VEGF antibody, an effective amount of a chemotherapeutic and an effective amount of radiotherapy, wherein said treatment prolongs said patient's median progression-free survival time as compared to a glioblastoma patient receiving said chemotherapeutic without an anti-VEGF antibody.

2. The anti-VEGF antibody for use of claim 1, wherein said patient has a WHO performance status of ≤2.

3. The anti-VEGF antibody for use of claim 1, wherein the chemotherapeutic is temozolomide.

4. The anti-VEGF antibody for use of clam 3, wherein the temozolomide is administered at 150 mg/m².

5. The anti-VEGF antibody for use of clam 3, wherein the temozolomide is administered at 200 mg/m².

6. The anti-VEGF antibody for use of claim 1, wherein said anti-VEGF antibody is bevacizumab.

7. The anti-VEGF antibody for use of claim 1, wherein said anti-VEGF antibody comprises a variable heavy chain (VH) and a variable light chain (VL), wherein said VH has an amino acid sequence of SEQ ID NO:2 and said VL has an amino acid sequence of SEQ ID NO: 1.

8. The anti-VEGF antibody for use of claim 1, wherein said effective amount of said anti-VEGF antibody is 10 mg/kg intravenously every two weeks.

9. The anti-VEGF antibody for use of claim 1, wherein said effective amount of said anti-VEGF antibody is 15 mg/kg intravenously every three weeks.

10. The anti-VEGF antibody for use of claim 1, wherein said effective amount of said anti-VEGF antibody is administered initially intravenously over 90 minutes, with subsequent infusions over 60 minutes and then 30 minutes.

11. The anti-VEGF antibody for use of claim 1, wherein said anti-VEGF antibody is administered first to said patient at the first cycle.

12. The anti-VEGF antibody for use of claim 8, wherein subsequent administrations of said anti-VEGF antibody are either prior to or after said chemotherapeutic.

13. The anti-VEGF antibody for use of claim 1, wherein said anti-VEGF antibody is administered concurrently with said chemotherapeutic.

14. The anti-VEGF antibody for use of claim 1, wherein said median progression-free survival time is prolonged by about 4.4 months with a hazard ratio (HR) equal to 0.64, as compared to a glioblastoma patient receiving said chemotherapeutic without the anti-VEGF antibody.

15. The anti-VEGF antibody for use of claim 1, wherein said median progression-free survival time is prolonged by at least 4 months or greater with a hazard ratio (HR) equal to 0.64, as compared to a glioblastoma patient receiving said chemotherapeutic without the anti-VEGF antibody.

16. The anti-VEGF antibody for use of claim 1, wherein said median progression-free survival time is prolonged by about 4.4 months with a hazard ratio (HR) from about 0.55 to about 0.74, as compared to a glioblastoma patient receiving said chemotherapeutic without the anti-VEGF antibody.

17. The anti-VEGF antibody for use of claim 1, wherein said median progression-free survival time is prolonged by at least 4 months or greater with a hazard ratio (HR) from about 0.55 to about 0.74, as compared to a glioblastoma patient receiving said chemotherapeutic without the anti-VEGF antibody.

18. The anti-VEGF antibody for use of any one of claims 14-17, wherein said patient is less than 65 years old.

19. The anti-VEGF antibody for use of any one of claims 14-17, wherein said patient is equal to or greater than 65 years old.

## Patentansprüche

1. Anti-VEGF-Antikörper zur Verwendung in einem Verfahren zur Behandlung eines Patienten, bei dem ein Glioblastom diagnostiziert wurde, wobei das Verfahren die Verabreichung einer Therapie an den Patienten umfasst, die eine wirksame Menge eines Anti-VEGF-Antikörpers, eine wirksame Menge eines Chemotherapeutikums und eine wirksame Menge an Strahlentherapie umfasst, wobei die Behandlung die mittlere progressionsfreie Überlebenszeit des Patienten im Vergleich zu einem Glioblastompatienten, der das Chemotherapeutikum ohne einen Anti-VEGF-Antikörper erhält, verlängert.

2. Anti-VEGF-Antikörper zur Verwendung nach Anspruch 1, wobei der Patient einen WHO-Performance-Status von ≤ 2 aufweist.

3. Anti-VEGF-Antikörper zur Verwendung nach Anspruch 1, wobei das Chemotherapeutikum Temozolomid ist.

4. Anti-VEGF-Antikörper zur Verwendung nach Anspruch 3, wobei das Temozolomid bei 150 mg/m² verabreicht wird.

5. Anti-VEGF-Antikörper zur Verwendung nach Anspruch 3, wobei das Temozolomid bei 200 mg/m² verabreicht wird.

6. Anti-VEGF-Antikörper zur Verwendung nach Anspruch 1, wobei der Anti-VEGF-Antikörper Bevacizumab ist.

7. Anti-VEGF-Antikörper zur Verwendung nach Anspruch 1, wobei der Anti-VEGF-Antikörper eine variable Schwerkette (VH) und eine variable Leichtkette (VL) umfasst, wobei die VH eine Aminosäuresequenz von Seq.-ID Nr. 2 aufweist und die VL eine Aminosäuresequenz von Seq.-ID Nr. 1 aufweist.

8. Anti-VEGF-Antikörper zur Verwendung nach Anspruch 1, wobei die wirksame Menge des Anti-VEGF-Antikörpers 10 mg/kg intravenös alle zwei Wochen beträgt.

9. Anti-VEGF-Antikörper zur Verwendung nach Anspruch 1, wobei die wirksame Menge des Anti-VEGF-Antikörpers 15 mg/kg intravenös alle drei Wochen beträgt.

10. Anti-VEGF-Antikörper zur Verwendung nach Anspruch 1, wobei die wirksame Menge des Anti-VEGF-Antikörpers zunächst intravenös über 90 Minuten verabreicht wird, mit darauffolgenden Infusionen über 60 Minuten und anschließend 30 Minuten.

11. Anti-VEGF-Antikörper zur Verwendung nach Anspruch 1, wobei der Anti-VEGF-Antikörper dem Patienten zuerst im ersten Durchgang verabreicht wird.

12. Anti-VEGF-Antikörper zur Verwendung nach Anspruch 8, wobei nachfolgende Verabreichungen des Anti-VEGF-Antikörpers entweder vor oder nach dem Chemotherapeutikum erfolgen.

13. Anti-VEGF-Antikörper zur Verwendung nach Anspruch 1, wobei der Anti-VEGF-Antikörper gleichzeitig mit dem Chemotherapeutikum verabreicht wird.

14. Anti-VEGF-Antikörper zur Verwendung nach Anspruch 1, wobei die mittlere progressionsfreie Überlebenszeit im Vergleich zu einem Glioblastompatienten, der das Chemotherapeutikum ohne den Anti-VEGF-Antikörper erhält, bei einer Hazard-Ratio (HR) von 0,64 um etwa 4,4 Monate verlängert wird.

15. Anti-VEGF-Antikörper zur Verwendung nach Anspruch 1, wobei die mittlere progressionsfreie Überlebenszeit im Vergleich zu einem Glioblastompatienten, der das Chemotherapeutikum ohne den Anti-VEGF-Antikörper erhält, bei einer Hazard-Ratio (HR) von 0,64 um zumindest 4 Monate oder mehr verlängert wird.

16. Anti-VEGF-Antikörper zur Verwendung nach Anspruch 1, wobei die mittlere progressionsfreie Überlebenszeit im Vergleich zu einem Glioblastompatienten, der das Chemotherapeutikum ohne den Anti-VEGF-Antikörper erhält, bei einer Hazard-Ratio (HR) von etwa 0,55 bis etwa 0,74 um etwa 4,4 Monate verlängert wird.

17. Anti-VEGF-Antikörper zur Verwendung nach Anspruch 1, wobei die mittlere progressionsfreie Überlebenszeit im Vergleich zu einem Glioblastompatienten, der das Chemotherapeutikum ohne den Anti-VEGF-Antikörper erhält, bei einer Hazard-Ratio (HR) von etwa 0,55 bis etwa 0,74 um zumindest 4 Monate oder mehr verlängert wird.

18. Anti-VEGF-Antikörper zur Verwendung nach einem der Ansprüche 14 bis 17, wobei der Patient weniger als 65 Jahre alt ist.

19. Anti-VEGF-Antikörper zur Verwendung nach einem der Ansprüche 14 bis 17, wobei der Patient gleich oder mehr als 65 Jahre alt ist.

## Revendications

1. Anticorps anti-VEGF destiné à être utilisé dans un procédé de traitement d'un patient présentant un diagnostic de glioblastome, où le procédé comprend l'administration audit patient d'une thérapie comprenant une quantité efficace d'un anticorps anti-VEGF, une quantité efficace d'un agent de chimiothérapie et une quantité efficace d'une radiothérapie, où ledit traitement prolonge la durée de survie médiane sans progression dudit patient par comparaison à un patient présentant un glioblastome recevant ledit agent de chimiothérapie sans un anticorps anti-VEGF.

2. Anticorps anti-VEGF destiné à être utilisé selon la revendication 1, où ledit patient présente un indice de performance OMS ≤ 2.

3. Anticorps anti-VEGF destiné à être utilisé selon la revendication 1, où l'agent de chimiothérapie est le témolozomide.

4. Anticorps anti-VEGF destiné à être utilisé selon la revendication 3, où le témolozomide est administré à 150 mg/m².

5. Anticorps anti-VEGF destiné à être utilisé selon la revendication 3, où le témolozomide est administré à 200 mg/m².

6. Anticorps anti-VEGF destiné à être utilisé selon la revendication 1, où ledit anticorps anti-VEGF est le bévacizumab.

7. Anticorps anti-VEGF destiné à être utilisé selon la revendication 1, où ledit anticorps anti-VEGF comprend une chaîne lourde variable (VH) et une chaîne légère variable (VL), où ladite VH possède une séquence d'acides aminés de SEQ ID NO: 2 et ladite VL possède une séquence d'acides aminés de SEQ ID NO: 1.

8. Anticorps anti-VEGF destiné à être utilisé selon la revendication 1, où ladite quantité efficace dudit anticorps anti-VEGF est 10 mg/kg par voie intraveineuse toutes les deux semaines.

9. Anticorps anti-VEGF destiné à être utilisé selon la revendication 1, où ladite quantité efficace dudit anticorps anti-VEGF est 15 mg/kg par voie intraveineuse toutes les trois semaines.

10. Anticorps anti-VEGF destiné à être utilisé selon la revendication 1, où ladite quantité efficace dudit anticorps anti-VEGF est initialement administrée par voie intraveineuse pendant 90 minutes, avec des perfusions ultérieures pendant 60 minutes puis 30 minutes.

11. Anticorps anti-VEGF destiné à être utilisé selon la revendication 1, où ledit anticorps anti-VEGF est administré en premier audit patient lors du premier cycle.

12. Anticorps anti-VEGF destiné à être utilisé selon la revendication 8, où les administrations ultérieures dudit anticorps anti-VEGF sont réalisées avant ou après ledit agent de chimiothérapie.

13. Anticorps anti-VEGF destiné à être utilisé selon la revendication 1, où ledit anticorps anti-VEGF est administré de manière concomitante avec ledit agent de chimiothérapie.

14. Anticorps anti-VEGF destiné à être utilisé selon la revendication 1, où ladite durée de survie médiane sans progression est prolongée d'environ 4,4 mois avec un rapport des risques (HR) égal à 0,64, par comparaison à un patient présentant un glioblastome recevant ledit agent de chimiothérapie sans l'anticorps anti-VEGF.

15. Anticorps anti-VEGF destiné à être utilisé selon la revendication 1, où ladite durée de survie médiane sans progression est prolongée d'au moins 4 mois ou plus avec un rapport des risques (HR) égal à 0,64, par comparaison à un patient présentant un glioblastome recevant ledit agent de chimiothérapie sans l'anticorps anti-VEGF.

16. Anticorps anti-VEGF destiné à être utilisé selon la revendication 1, où ladite durée de survie médiane sans progression est prolongée d'environ 4,4 mois avec un rapport des risques (HR) d'environ 0,55 à environ 0,74, par comparaison à un patient présentant un glioblastome recevant ledit agent de chimiothérapie sans l'anticorps anti-VEGF.

17. Anticorps anti-VEGF destiné à être utilisé selon la revendication 1, où ladite durée de survie médiane sans progression est prolongée d'au moins 4 mois ou plus avec un rapport des risques (HR) d'environ 0,55 à environ 0,74, par comparaison à un patient présentant un glioblastome recevant ledit agent de chimiothérapie sans l'anticorps anti-VEGF.

18. Anticorps anti-VEGF destiné à être utilisé selon l'une quelconque des revendications 14 à 17, où ledit patient est âgé de moins de 65 ans.

19. Anticorps anti-VEGF destiné à être utilisé selon l'une quelconque des revendications 14 à 17, où ledit patient est âgé de 65 ans ou plus.


### Sequence Listing

<110> Genentech, Inc. et al.
<120> Combination Therapy For the Treatment of Glioblastoma
<130> P4946R1-WO
<141> 2013-08-06
<150> US 61/760,763 US 61/714,438 US 61/680,672
<151> 2013-02-05 2012-10-16 2012-08-07
<160> 2
<210> 1
   <211> 108
   <212> PRT
   <213> Artificial sequence
<220>
   <223> sequence is synthesized
<400> 1
   Asp Ile Gln Met Thr Gln Ser Pro Ser Ser Leu Ser Ala Ser Val
     1               5                  10                  15
   Gly Asp Arg Val Thr Ile Thr Cys Ser Ala Ser Gln Asp Ile Ser
                    20                  25                  30
   Asn Tyr Leu Asn Trp Tyr Gln Gln Lys Pro Gly Lys Ala Pro Lys
                    35                  40                  45
   Val Leu Ile Tyr Phe Thr Ser Ser Leu His Ser Gly Val Pro Ser
                    50                  55                  60
   Arg Phe Ser Gly Ser Gly Ser Gly Thr Asp Phe Thr Leu Thr Ile
                    65                  70                  75
   Ser Ser Leu Gln Pro Glu Asp Phe Ala Thr Tyr Tyr Cys Gln Gln
                    80                  85                  90
   Tyr Ser Thr Val Pro Trp Thr Phe Gly Gln Gly Thr Lys Val Glu
                    95                 100                 105
   Ile Lys Arg
<210> 2
   <211> 123
   <212> PRT
   <213> Artificial sequence
<220>
   <223> sequence is synthesized
<400> 2